# EUROPÄISCHE PATENTANMELDUNG

(11) **EP 2 581 046 A1**
(43) Veröffentlichungstag der Anmeldung: **17.04.2013**
(21) Anmeldenummer: 12188670.9
(22) Anmeldetag: 16.10.2012
(51) Int. Cl.: A61B 17/04, A61F 2/08

(54) **Anker zur Befestigung von Gewebe im Knochen**

(30) Priorität: 16.10.2011 CH 16832011
(71) Anmelder: Da Rold, Orlando, 4500 Solothurn (CH)
(72) Erfinder: Da Rold, Orlando, 4500 Solothurn (CH)
(74) Vertreter: Fleck, Hermann-Josef

(57) **Zusammenfassung**

Es werden Schraub- und Stossanker vorgestellt, deren Lappen 3 vier Mitnahmeflächen 6 aufweisen, die der Führung und dem Angriff von Instrumenten dienen. Die Oberflächen des Lappens sind fein bearbeitet, so dass ein darüber laufender Faden durch eine Öffnung leicht durchgezogen werden kann und keinen Schaden leidet. Die spezielle geometrische Form, insbesondere die Führung der Instrumente an den präzise gearbeiteten Mitnahmeflächen 6 ermöglicht saubere und effiziente Arbeit des Operateurs für minimal invasive Eingriffe. Speziell die Möglichkeit, die korrekte Spannung der Fäden einzustellen hilft dabei mit, dass sich die vom Körper anzupassenden Bänder, Sehnen und Muskelansätze schneller und besser an die Postoperative Situation anpassen.

## Beschreibung

Die vorliegende Erfindung betrifft Anker zur Befestigung von Gewebe in Knochen gemäss Oberbegriff des Patentanspruchs 1.

Anker für die Befestigung von Gewebe mittels Verankerung der Fäden z.B. bei Schulteranwendungen, wie sie üblich sind für die Rekonstruktion der Rotatorenmanschette, für Blankart- und SLAP-Läsionen, für Bizepstenodese, für die Reparatur des Deltamuskels und für Kapselshift oder Rekonstruktion der Kapselränder. Ebenso wird diese Art der Befestigung unter anderem für die laterale und mediale Stabilisierung an Fuss und Knöchel oder für die Reparatur der Achillessehne verwendet. Anker sind vor allem für minimal invasive Operations-Techniken ideal geeignet und im Einsatz.

Solche Anker werden immer als Set mit zugehörigen Instrumenten, in der Form von sterilen Einheiten zur Verfügung gestellt. Man findet ähnliche Sets dieser Art z.B. im Internet auf http://www.smith-nephew.de/produkte/. In der Regel wir der sorgfältigen Entwicklung und vor allem auch der sorgfältigen Planung des mechanischen Herstellungsprozesses des Ankers zu wenig Aufmerksamkeit geschenkt. Dies mag dadurch erklärbar sein, dass der medizinische Teil der Zulassung und die Vorgehensweise der Faden Anordnung und Knotung für den Erfolg einer minimal invasiven Chirurgie grosse Aufmerksamkeit erheischen und dadurch im zentralen Interesse der Hersteller und Operateure sind. Der mechanischen Herstellung der Hilfsmittel wie Anker und Instrumente für deren präzises Einbringen wird zu wenig Aufmerksamkeit gewidmet. Dies ist bedauerlich, ist doch die optimale Form der Anker für das Einbringen, den Halt im Knochengewebe, die Qualität der Fixierung und den gesamten Erfolg des Eingriffs und ganz besonders für die erfolgreiche Rehabilitation des Patienten entscheidend. Dieser Forderung werden auf dem Markt vorhandene Anker nicht genügend gerecht. Man begnügt sich damit, dass man mit den herkömmlichen Produkten statistisch positive Erfolge belegen kann. Das Einzelschiksal und insbesondere die Dauer der Rehabilitation der Patienten spielt eine untergeordnete Rolle.

Die vorliegende Erfindung stellt sich die Aufgabe einen Anker zur Befestigung von Gewebe in Knochen der eingangs genannten Art derart zu verbessern, dass die Vorteile der bekannten minimal invasiven Chirurgie erhalten bleiben, der Anker zur Befestigung von Gewebe in Knochen jedoch einfacher und effizienter zu handhaben ist, sich im Knochengewebe besser verankert und damit die Rehabilitation des Patienten signifikant verkürzt.

Diese Aufgabe lösen Anker zur Befestigung von Gewebe in Knochen mit den Merkmalen des Patentanspruches 1. Weitere erfindungsgemässe Merkmale gehen aus den abhängigen Ansprüchen hervor und deren Vorteile sind in der nachfolgenden Beschreibung erläutert.

In der Zeichnung zeigt:
- Fig 1: Bild eines Stossankers.
- Fig 2: Perspektivische Ansicht eines Stossankers.
- Fig 3: Schnitt eines Stossankers mit dem Instrument.
- Fig 4: Seitenansicht eines Stossankers.
- Fig 5: Aufsicht eines Stossankers.
- Fig 6: Aufsicht auf den Lappen eines Stossankers.
- Fig 7: Bild eines Schraubankers.
- Fig 8: Perspektivische Ansicht eines Schraubankers.
- Fig 9: Seitenansicht eines Schraubankers.
- Fig 10: Aufsicht eines Schraubankers mit Schnitt durch den Lappen.
- Fig 11: Aufsicht auf den Lappen eines Schraubankers.
- Fig 12: Schraubanker mit Drehinstrument.
- Fig 13: Aufsicht auf den Lappen eines Schraubankers.

Die Figuren stellen mögliche Ausführungsbeispiele dar, welche in der nachfolgenden Beschreibung erläutert werden.

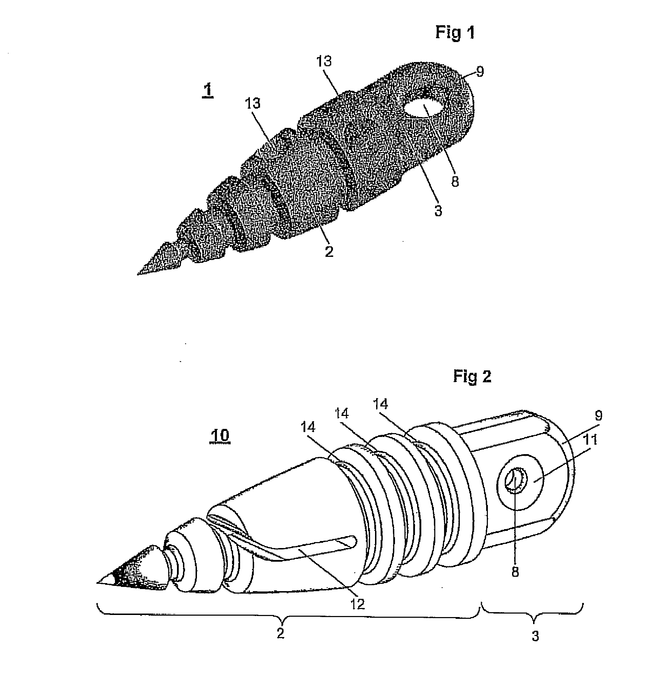

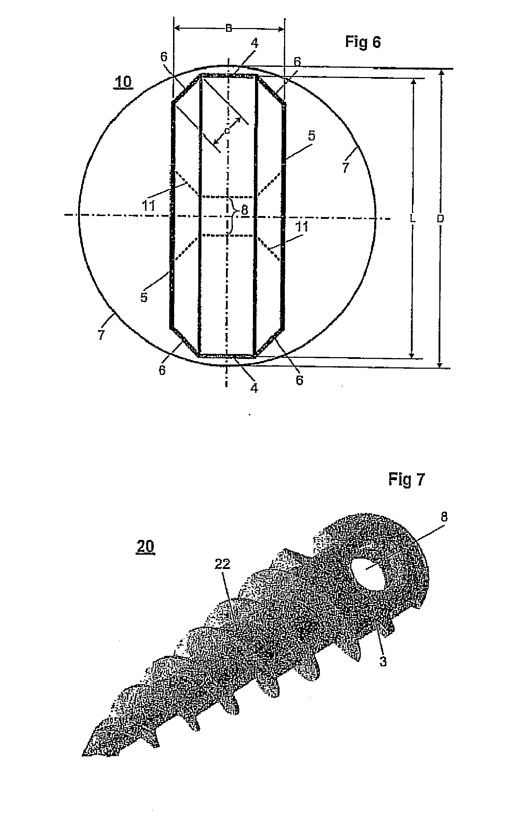

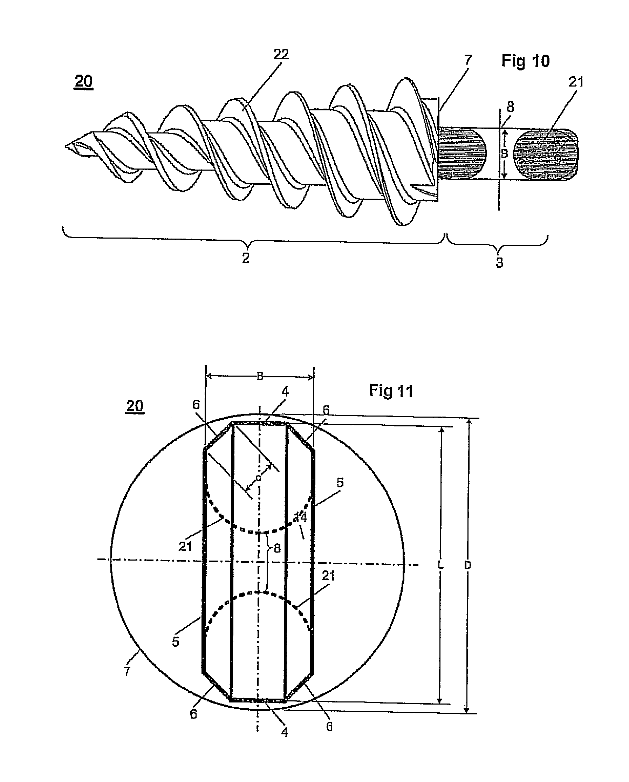

Anker zur Befestigung von Gewebe in Knochen sind seit längerem bekannt und werden für minimal invasive Operationen häufig und erfolgreich eingesetzt. Sie bestehen aus einem Hauptkörper 2 (Fig 1 & 2) der eine runde Form aufweist und sich von einem spitzen proximalen Ende in einer leicht nach aussen gekrümmten konischen Form verläuft, um an einer Kante 7 (Fig 2, 5 & 10) mit dem grössten Durchmesser D zu enden. Die Fortsetzung am proximalen Ende bildet ein Lappen 3. Dieser Lappen bildet von der Ansicht vom proximalen Ende aus gesehen (Fig 6 und Fig 11)ein unregelmässiges Achteck. Dieses Achteck ist begrenzt durch zwei Längsflächen 5, zwei Breitflächen 4 und vier Mitnahmeflächen 6. Diese Mitnahmenflächen 6 sind Führungsflächen, an welchen ein Instrument 17 für Stossanker 10 (Fig 2-6) und ein Drehinstrument 27 für Schraubanker 20 (Fig 7-11) angreifen. Gegenüber herkömmlichen Lappen mit Sechskant bietet diese Form den wichtigen Vorteil, eine optimalere und präzisere Fass- und Angreifmöglichkeit für das jeweilige Instrument. Entscheidend für den erfolgreichen und effizienten Einsatz solcher Anker ist die präzise Führung der Stoss- und Schraubanker 10,20 während des Einsetzens.

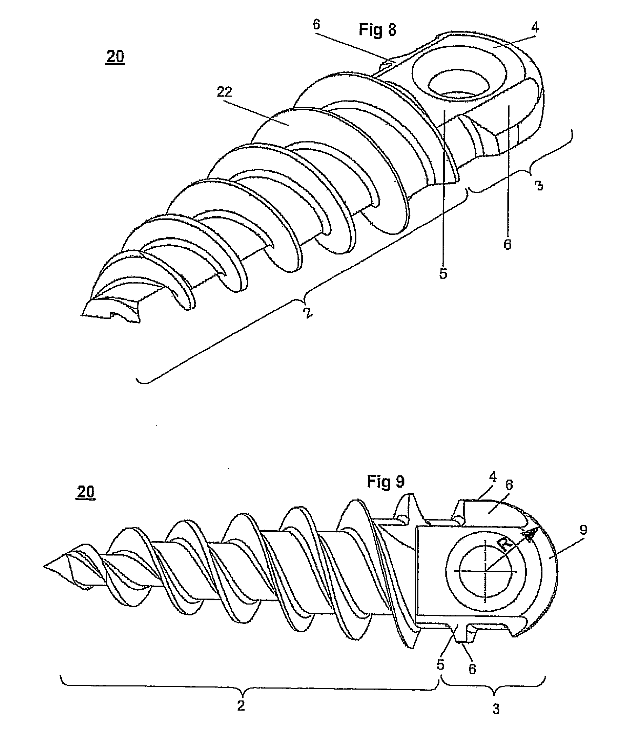

Um den Lappen 3 mit dem Instrument 17 für den Stossanker 10 oder mit dem Drehinstrument 27 für den Schraubanker 20 sicher treffen zu können, ist der Lappen am proximalen Ende mit einer Rundung mit dem Radius R (Fig 9) versehen. Im praktische Sinne stellt der Lappen 3 eine Öse dar, welche aber für Stossanker 10 respektive Schraubanker 20 verschiedenen, funktional spezielle Formen aufweist.

Ein entscheidendes Kriterium für den erfolgreichen Einsatz von Stoss- und Schraubanker 10,20 ist die Möglichkeit je nachdem während, oder nach dem setzen eines Stoss- oder Schraubankers 10,20, die Fäden in die für den Halt der Sehnen, Bänder und Muskeln richtige Spannung versetzen zu können. Zwar wird sich das Gewebe postoperativ durch Physiotherapie der Spannung der Suturen anpassen, jedoch geht diese Restitution des Zusammenspiels der Verankerung und des Gewebes schneller, je besser und optimaler diese Spannung der Fäden für die Situation richtig gewählt wurde. Man verwendet deshalb je nach Bedarf sogenannte Stossanker 10 oder Schraubanker 20.

Im praktischen Einsatz wird man zum Beispiel den Faden am einen Ende des zu fixierenden Bandes, Muskels oder der zu fixierenden Sehne befestigen und mit einem Stossanker 10 fixieren. Die korrekte Position des Fadens wird durch den Ort an dem der Stossanker 10 gesetzt wird bestimmt. Dieser Faden ist dann im Stossanker 10 unverrückbar fixiert. Im Bereich des andern Endes des zu fixierenden Bandes, Muskels oder der zu fixierenden Sehne wird sodann ein Schraubanker 20 gesetzt. Ein Faden wird sodann zwischen dem Band, Muskel oder der Sehne und dem gesetzten Schraubanker 20 eingefädelt und verknüpft, so dass das zu fixierenden Band, der Muskel oder die zu fixierende Sehne die gewünschte Spannung erhält. Für diesen Vorgang muss der Faden in der Öffnung 8 der Ringform 21 des Schraubankers 20 (Fig 10) beweglich sein und darf beim Nachziehen keinen Schaden nehmen. Für Schraubanker 20 ist es deshalb wünschenswert ja sogar erforderlich, dass man die Fäden auch nach dem Setzen des Schraubankers 20 noch bewegen kann ohne dass sie am Ring verletzt werden.

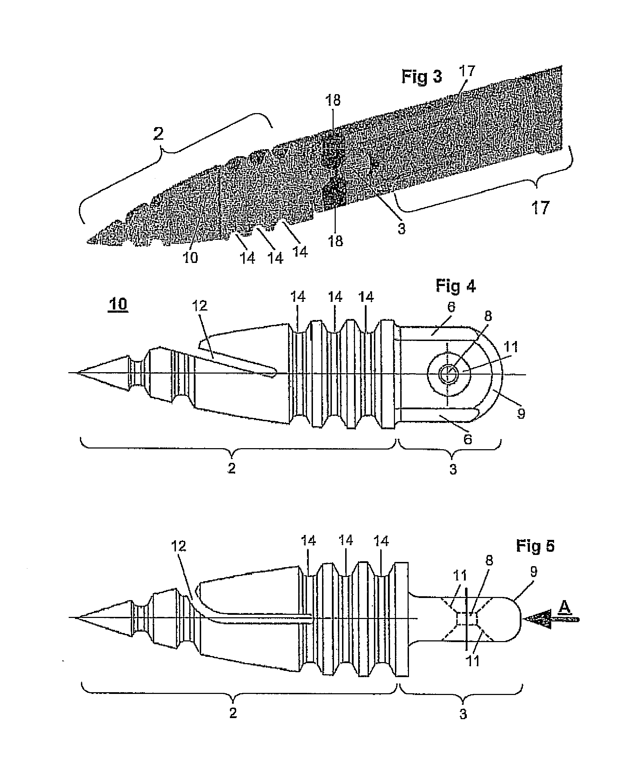

Für Stossanker 10 ist es jedoch wesentlich, dass dieser mit einem Instrument 17 (Fig 3) für das Einsetzen fest gehalten werden und im Bedarfsfall mit diesem Instrument 17 auch wieder entfernt werden kann. Ein Stossanker 10 weist deshalb eine Bohrung 8 auf, die mit einem Krater 11 versehen ist (Fig 6). Das Instrument 17 nimmt den Lappen 3 formschlüssig auf (Fig 3) und die federnd gelagerten Knöpfe 18 halten den Stossanker 10 indem sie im Krater 11 im Lappen 3 einrasten. Wenn der Stossanker 10 gesetzt und im Knochen verankert ist, kann das Instrument 17 heraus gezogen werden, da die federnde Lagerung der Knöpfe 18 ein Hinausgleiten über den Krater 11 zulässt.

Muss unvorhergesehen ein Stossanker 10 im Bedarfsfall wieder entfernt werden, wird hinten am Schaft über das Instrument 17 ein Rohr geschoben. Das Instrument 17 wird dann erneut über den Lappen 3 des Stossankers 10 gebracht, bis die Knöpfe 18 einrasten. Dann wird das Rohr nach vorne geschoben, damit die federnde Lagerung der Knöpfe 18 aufgehoben und damit der Lappen 3 vom Instrument 17 fest gehalten. Nun kann der Stossanker 10 hinrausgezogen werden.

Viele bekannte Stossanker 10 weisen Bohrungen 13 (Fig 1) auf, durch welche die Fäden vor dem Setzen eines Stossankers 10 durchgezogen werden. Im eingesetzten Zustand ist die Schlaufe des Fadens dann in einer solchen Bohrung 13 zwischen Anker und Knochengewebe unverrückbar gehalten. Der doppelt oder dreifach geführte Faden ist mit dem zu befestigenden Gewebe verbunden. Die in dieser Schrift vorgestellten Stossanker 10 weisen demgegenüber einen Schlitz 12 für dieselbe Funktion auf (Fig 2, 4 und 5). Vorteil dieses Schlitzes ist das sehr viel einfachere "Einfädeln" des Fadens in einen Schlitz dieser Art.

Der bereits beschriebene Schraubanker 20 wird dazu verwendet, an Ort und Stelle die erforderliche Fadenlänge einzustellen. Dafür sind die Radien der Ringform 21 und der Rundform 9 (Fig 9 & 11), sowie die Ausbildung der Oberflächenfeinheit dieser Radien sind massgebend dafür, ob die Fäden leicht nachgezogen und damit in die für die Situation richtige Spannung versetzt werden können. Weist die Öffnung 8 z.B. scharfe oder nicht sauber bearbeitete Riefen auf, so ist das Nachziehen der Fäden schwierig und der Faden könnte Schaden nehmen.

Erschwerend für die Herstellung optimaler Öffnungen 8 ist der Umstand, dass ein solcher Anker 1 (Fig 1) sehr klein ist. Im Regelfall weist ein Anker 1 eine Länge von ca. 10 mm und einen grössten Durchmesser von ca. 2.5 bis 5.5mm auf. Die präzise mechanische Bearbeitung der Mitnahmeflächen 6 des Lappens 3 und der Ringform 21 der Öffnung 8 ist deshalb schwierig, aufwendig und sehr speziell, jedoch absolut erforderlich.

Für das Verständnis der ganzen Vorrichtung soll im folgenden die Bereitstellung im Operationssaal beschrieben werden:

Ein Set bestehend aus Instrumenten, Zubehör und voraussichtlich zu verwendenden Anker wird steril zur Verfügung gestellt, so dass die Operationsschwester alles für das Setzen des Ankers vorbereiten kann. Im Instrumentenset befinden sich nebst Instrument 17 und Drehinstrument 27 auch Vorrichtungen mit welchen die Operationsschwester bequem - und deshalb zuverlässig die Fäden in Stossanker 10 und Schraubanker 20 einfädeln kann.

Die Stossanker 10 und die Schraubanker 20 sind, jeder für sich mit einer Hülle versehen. Dies, damit sie von der Operationsschwester besser gehalten werden und die Spitzen von Schraub- und Stossanker sowie die scharfen Kanten der Gewinde der Schraubanker bei der Bereitstellung der Vorrichtung, nicht die Gummihandschuhe des Operationsteams verletzt. Anderseits wird damit gewährleistet, dass die Stoss- und Schraubanker bis zum Einsatz absolut steril bleiben. Erst unmittelbar vor dem Einführen der Anker an der Operationsstelle, wird diese Hülle entfernt.

Um den Faden beim Einführen fixieren zu können, kann dem Hauptkörper 1 eines Stossankers 10 seitlich von der Spitze weg etwa im ersten Viertel des Konus beginnend und in einer beliebigen Kurve zum Zentrum des Ankers hin verlaufender Schlitz 12 (Fig 2,4 & 5) angebracht werden. In diesem Schlitz 12 können z.B. erste Fäden absolut fest verankert mit dem Stossanker zusammen im Knochen eingebracht werden.

Am konischen Teil, also am Hauptkörper 2 eines Stossankers 10 (Fig 2-6) sind trapezförmige Einstiche 14 angebracht, welche das Ausreissen des Stossankers aus dem Knochengewebe vermeiden helfen. Ziel ist es, das schwammige Gewebe der vom Körper nach einem Eingriff erfolgende Kallus Bildung in diese Rillen eintreten zu lassen und damit dem Anker 1 guten Halt im Knochen zu geben. Gleichzeitig werden die Fäden um die trapezförmigen Einstiche 14 umgelenkt und fest gehalten. Man vermeidet damit automatisch das Lösen und Gleiten der Fäden.

Beim Schraubanker 20 (Fig 7 -11) wird am Konus ein mindestens eingängiges Gewinde angebracht. Durch das Einschrauben eines Schraubankers 20 wird dieser im Knochen wie eine Schraube verankert. Zusammen mit der später erfolgenden Kullus Bildung wird der Halt noch besser. Schraubanker werden dann mit Vorteil eingesetzt, wenn mit distalen Kräften der Fäden auf den Anker zu rechnen ist.

Für den einfachen und bequemen Einsatz und dadurch für den Erfolg der Operation entscheidend ist die Ausbildung des Lappens 3. Zum einen muss er dem Stossanker 10 respektive dem Schraubanker 20 während des Einsetzens mit dem Schraubenzieher ähnlichen Instrument 17 für den Stossanker 10 und dem Drehinstrument für den Schraubanker sicher gehalten werden können. Zum andern muss er einmal an Ort eingesetzt ohne grossen Kraftaufwand leicht vom Instrument 20 resp. Drehinstrument gelöst werden können.

Um von einem Instrument 20 (Fig 3) für einen Stossanker 10 oder einem Drehinstrument für Schraubanker 20 gefasst sondern und in die Position gestossen oder gedreht werden zu können, muss der Lappen 3 eine gewisse Festigkeit aufweisen, damit er die über das Instrument 20 oder das Drehinstrument eingebrachte Kraft auf den Hauptkörper 2 des Ankers übertragen kann, ohne abzubrechen. Die Länge L (Fig 6,11 des Lappens 3 sollte so gross wie möglich, damit auf den Hauptkörper 2 ein wirkungsvolles Drehmoment übertragen werden kann. Man wird danach trachten, die Länge L so nahe wie möglich an die Dimension des grössten Durchmesser D des Hauptkörpers 2 zu bringen. Die Länge L wird jedoch immer kleiner sein, als der grösste Durchmesser D, damit eine Kante 7 entsteht, welche durch die Kallus Bildung zum Halt eines Ankers 1 im eingebrachten Zustand beiträgt.

Die Breite B (Fig 6,11) des Lappens 3 muss ebenfalls eine stabile Dimension aufweisen. Idealerweise wird für einen Schraubanker 20 die Breite B so gewählt, dass am distalen Ende des Lappens 3 durch die Rundung und die Rundform 9 des Lappens und die Ringform 21 der Öffnung 10 ein halber Ring mit dem Durchmesser B (Fig 10) entsteht. Abweichungen von diesem Idealzustand sind nicht zu vermeiden. Wenn die Oberflächen der Ringform 21 und der Rundform 9 jedoch fein bearbeitet sind, können die Fäden auch nachgezogen werden, wenn die Form eines Ringes (Fig 10) mit Durchmesser B am distalen Ende des Lappens 3 nur angenähert erreicht wird.

Die Länge L (Fig 6,11) wird durch die Breitflächen 4 und die Breite B wird durch die Längsflächen 5 begrenzt. An den Ecken, wo sich Breitflächen 4 und Längsfläche 5 treffen sind Mitnahmeflächen 6 angebracht. Aus der geometrischen Form ergibt sich die Definition, dass die Längsflächen 5 länger sind als die Breitflächen 4, und letztere wieder länger sind als die Mitnahmeflächen 6 (Fig 6,11). Die Mitnahmeflächen 6 weisen eine Breite C von 0.1mm bis 2.0mm auf. Die Mitnahmeflächen 6 verlaufen vom proximalen Anfang des Lappens 3 bis zum distalen Übergang auf die Rundform 9. Am distalen Ende der Mitnahmeflächen 6 gehen diese über in die Rundform 9 (Fig 8), welche distal den Lappen 3 abschliessen.

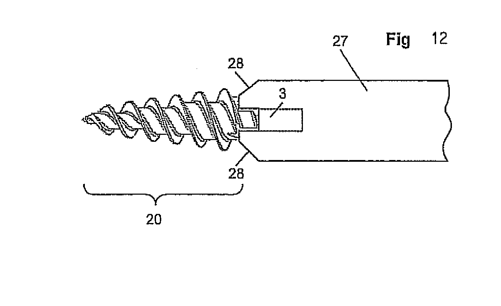

Das für das Eindrehen eines Schraubankers 20 verwendete Drehinstrument 27 ist im Durchmesser grösser als der grösste Durchmesser D des Schraubankers 20 (Fig 12). Es weist am proximalen Ende eine Anfasung 28 auf, so dass während des Eindrehens um den Anker herum eine Krater ähnliche Öffnung entsteht. Diese ermöglicht während der Operation leichteren Zugriff auf den Schraubanker 20, leichteres Nachziehen des Fadens und vermeidet zudem während der Heilung ein unerwünschtes starkes Einschneiden im Knochen, da der Faden über Ränder der Krater ähnlichen Öffnung getragen wird und nicht nur an einer Kante angreift, bzw. sich in einer Kante im Knochen einfrisst.

## Patentansprüche

1. Anker zur Befestigung von Gewebe in Knochen,
**dadurch gekennzeichnet,**
**dass** ein runder Hauptkörper (2) vom proximalen spitzen Ende konisch zu einem distalen Ende und einem grössten Durchmesser (D) mit einer scharfen Kante (7) endet, wobei auf der entstehenden Fläche ein Lappen (3) aufgebaut ist, welcher innerhalb des grössten Durchmessers (D) des Hauptkörpers (2) ein zentral angeordnetes unregelmässiges Achteck bildet, dessen Grundriss aus zwei Breitflächen (4), zwei Längsflächen (5) und vier Mitnahmeflächen (6) besteht, wobei die Längsflächen (5) mindestens die Länge der Breitflächen (4) aufweisen und die Breitflächen (4) mindestens die Länge der Mitnahmefiächen (6) aufweisen, wobei der Lappen (3) mindestens eine zu den Längsflächen (5) rechtwinklig angeordnete, die Breite (B) des Lappens (3) durchdringende Öffnung (8) aufweist.

2. Anker nach Anspruch 1, **dadurch gekennzeichnet, dass** die Breite (C) der Mitnahmeflächen (6) 0.1mm bis 2.0mm aufweist.

3. Anker nach Anspruch 1, **dadurch gekennzeichnet, dass** der Lappen (3) am distalen Ende als Halbrund mit einem Radius R und einer Rundform (9) endet.

4. Anker nach Anspruch 1, **dadurch gekennzeichnet**, das die Mitnahmeflächen (6) am distalen Ende im Bereich der Rundform (9) auslaufen.

5. Anker nach Anspruch 1, **dadurch gekennzeichnet, dass** die Öffnung (8) mit den Längsflächen (5) eine Ringform (21) aufweist.

6. Anker nach Anspruch 1, **dadurch gekennzeichnet, dass** die Mittellinie der Öffnung (8) zur Längsfläche (5) senkrecht steht.

7. Anker nach Anspruch 1, **dadurch gekennzeichnet, dass** der Hauptkörper (2) einen vom Aussendurchmesser von einer proximal beginnenden Position distal schräg zum Zentrum hin verlaufenden Schlitz (12) aufweist.

8. Anker nach Anspruch 1, **dadurch gekennzeichnet, dass** der Hauptkörper (2) an der konischen Aussenseite ein Gewinde (22) aufweist.

9. Anker nach Anspruch 8, **dadurch gekennzeichnet, dass** das Gewinde (22) des Hauptkörpers (2) selbstschneidend ist.

10. Anker nach Anspruch 1, **dadurch gekennzeichnet, dass** senkrecht zur Achse des Hauptkörpers (2) der konische Teil, trapezförmige Einstiche (14) aufweist.
